# EUROPEAN PATENT SPECIFICATION

(11) **EP 2 965 776 B1**
(45) Date of publication and mention of the grant of the patent: **22.07.2020**
(21) Application number: 15173517.2
(22) Date of filing: 24.06.2015
(51) Int. Cl.: A61M 16/00, A61M 16/06, A61M 16/08, A61B 5/087, A63B 23/18, A63B 21/06, A63B 71/06

(54) **DEVICE FOR IMPROVING NASAL VENTILATION**
Vorrichtung zur Verbesserung der Nasenbeatmung
Dispositif pour améliorer la respiration nasale

(30) Priority: 25.06.2014 IT MI20141153
(43) Date of publication of application: 13.01.2016
(73) Proprietor: Milesi, Mario, 24060 Brusaporto (Bergamo) (IT)
(72) Inventor: Milesi, Mario, 24060 Brusaporto (Bergamo) (IT)
(74) Representative: Lunati & Mazzoni S.r.L.

(56) References cited:
- US-A- 4 232 683
- US-A- 4 579 124
- US-A- 5 042 478
- US-A1- 2002 046 755
- US-B1- 6 238 353

## Description

The present invention relates to a device for improving nasal ventilation of the type described in the preamble of the first claim.

This invention is not a diagnostic tool but a therapeutic aid. It should be used by patients for whom the diagnosis of absent or insufficient nasal ventilation has been clinically ascertained and who thus require a correction thereof.

In fact, as is well known in the medical field, breathing through the nose and not through the mouth is very important for various aspects and significantly affects a person's general state of health.

In particular, breathing through the nose heats, humidifies and filters the air and enriches it with nitrogen monoxide (NO).

The conditioning of air with nitric oxide (broncho and vasodilator) optimises the gaseous exchange at the level of the pulmonary alveoli by increasing the percentage of oxygen in the blood. The lack of this effect combined with the failure to cool the cerebral base when the airflow through the nose and nasopharynx is absent, determines a lower IQ in subjects breathing through the mouth. Such lower IQ has been shown both by clinical trials as well as being common knowledge: people who keep their mouths open at rest are certainly not deemed "bright" in public opinion.

Nitric oxide also has a defensive effect against bacteria, viruses and fungi. Its absence or reduction, along with the reduction of air flow is the cause of stagnation in the nasal passages of mucus with micro-organisms and possible diseases such as: rhinitis, sinusitis, tonsillitis, adenoiditis, otitis, the formation of polyps, etc..

Conversely, breathing through the mouth does not filter, humidify, warm or enrich the air specifically with nitric oxide.

The gaseous exchange at a pulmonary level is therefore not optimal, with obvious consequences on the entire body of a partial deficiency of oxygenation.

Another pathological consequence of absent or shortened nasal ventilation is the diminished enlargement (pneumatization) of the paranasal sinuses. With normal/physiological nasal ventilation at each breath the exhaled air is in part pushed, by the anatomical conformation of the upper airways, into the sinuses to then be sucked in, enriched with nitric oxide, while inhaling. It is this continuous "pumping" in the paranasal sinuses with each breath that determines during the years of growth of every individual the physiological enlargement of the sinuses and in particular the formation/pneumatization of the frontal paranasal sinus, from 6-8 years old, which unlike the others, is not present at birth. Often individuals with insufficient nasal ventilation show a "flat face" and in these subjects radiographic detection of a hypopneumatization or total absence of the frontal paranasal sinus is very common. All this results in a reduced production and release of nitrogen monoxide into the respiratory system and the orthognatodontic effects due to hypoplasia of the intermediate part of the skull.

Breathing through the mouth may occur at all ages, but can be found in children in particular when obstruction of the nasal ventilation airways, even temporary or already resolved surgically, have led to the development of an incorrect pattern of oral respiration that persists despite an obstacle to proper ventilation no longer being present. This is what sometimes happens, for example, in subjects who despite having undergone the removal of the adenoids, i.e. of the obstacle most often implicated in obstruction of nasal ventilation, continue to breathe through the mouth. These children, when asked to blow their noses on a handkerchief since suffering from rhinitis, actually blow from the mouth. In these cases functional therapy leading to the awareness of what nasal ventilation is and how to increase it to peak efficiency is important.

The simplest intervention, but necessarily sporadic and de facto invasive, is to impose keeping the mouth closed or, even more invasively, to cover the mouth with a plaster. Such intervention, unpleasant and stressful especially for children, can only temporarily achieve nasal breathing, during its performance, since it does not induce the brain to process the brain motor engrams for the upper airways making a nasal breathing pattern habitual.

Other devices exist in completely different sectors, but that can be considered structurally similar. This is the case of the device described in the US patent 4 579 124. This device is used to improve the diction and voice and has therefore nothing to do with the improvement of nasal breathing, nor is said device suitable to measure a flow rate similar to those of the nasal flow rates while exhaling at the maximum intensity possible by the patient.

In this situation the technical purpose of the present invention is to make a device for improving nasal ventilation able to substantially overcome the inconveniences mentioned above.

Within said technical task it is an important object of the invention to make a device such as to allow energetic and effective exercises, capable of solving the problems of incorrect breathing.

Another important object of the invention is to make a device which makes it possible to concentrate the exercises on a single nostril at a time to ensure that the processing of the proper breathing pattern is also accompanied by a marked thrust of the air exhaled in the paranasal sinuses to then be able to breathe it in from them greatly enriched with nitric oxide.

A further important object of the invention is to make a device for pleasant and stress-free use and suitable to be used frequently and constantly even by children. Another object of the invention is to make a device which allows those who use it, and the parents of young patients, to directly assess the results, the level of ventilation reached and the improvements achieved over time.

A further object of the invention of no less importance is to provide a device which allows a maximum hygiene and thorough cleaning of all its elements.

Said technical purpose and specified aims are achieved by a device as claimed in the appended claim 1.

Preferred embodiments are evident from the dependent claims.

The characteristics and advantages of the invention are clearly evident from the following detailed description of preferred embodiments thereof, with reference to the accompanying drawings, in which:
- **Fig. 1** shows a specific implementation of the device suitable to achieve maximum simplicity;
- **Figures 2a, 2b** show in side view and in transversal cross-section an embodiment of a sealing element or lancehead of the device;
- **Fig. 3** shows a portion of a device according to the invention fitted with two sealing elements of the type shown in the previous figures;
- **Fig. 4** shows a sealing element defined by a so-called nasal olive for nasal irrigation;
- **Fig. 5** is a variant of the device shown in Fig. 1 and
- **Fig. 6** shows a portion of the device including a further variant of the device in Fig. 1, which can be added to the device of figure 1.

With reference to the figures, the device according to the invention for improving nasal ventilation and therefore breathing is globally denoted by reference numeral **1.**

It is not used, nor can be used to improve speech, or diction and/or voice or for similar uses.

It includes, briefly, first means **2** suitable to collect a flow of air emitted by at least one nasal cavity and second means **3** suitable to highlight said airflow.

The second means 3 are suitable to highlight an airflow of a rate to the order of magnitude equal to the maximum flow rate in output from a nostril. This flow rate is much greater than the flow rate of air coming out of the nose for people affected by problems during speech.

The second means are not therefore used to measure the flow of air during speech.

More in detail, the first means 2 preferably comprise a sealing element inside a nostril **4** and a cannula **5.**

The sealing element 4 is designed to achieve a substantial seal between the nostril and the environment external to the cannula 5, for an air flow equal to the maximum flow rate out of one nostril, so that the air conveyed by said nostril ends up mainly inside the cannula 5. Of course the substantial seal, not being performed on mechanical elements but on nostrils with natural irregularities, will always be a limited seal. The term seal, in this case means that the air passes at least mainly in the cannula 5, or, more preferably in a quantity exceeding 75%. The sealing element 4 may have an arrow or lance or spearhead shape and is a body having externally a tapered outline or surface **6** suitable to permit its insertion in conditions of adherence into a nostril and having internally a through cavity **7** substantially aligned with the nostril in which it is inserted. The sealing element 4 also has, preferably, the diameter of the widest part of the alar rim of the nostril threshold. The insertion is provided for in a first, immediately accessible area of a portion of nostril called vestibule and threshold.

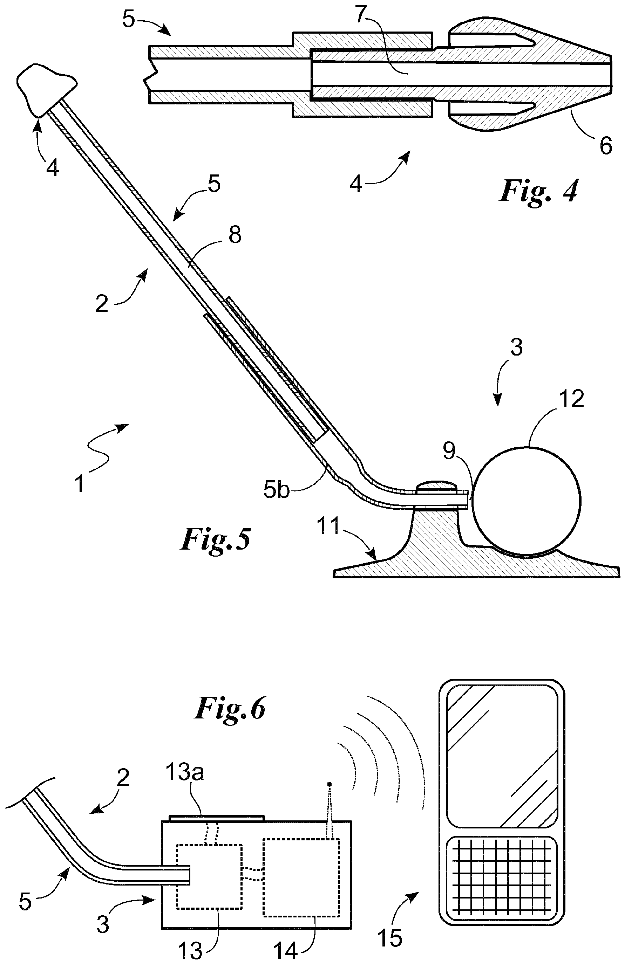

In one embodiment the sealing element 4 is a so-called nasal olive, shown in Fig. 4, in itself known inasmuch as used for nasal irrigation.

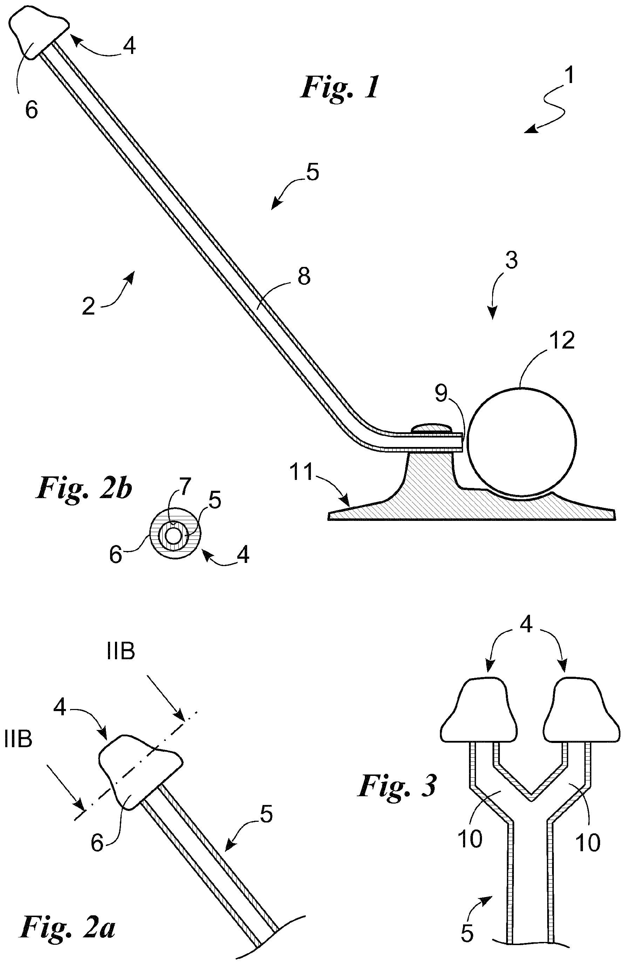

In a further embodiment, highlighted particularly in Figs. 2a, 2b, the sealing element 4 has an asymmetric shape corresponding to the asymmetric shape of each nostril, on one side delimited by the nasal septum and on the other part expanded in correspondence with an ala nasi.

It is preferably made of silicone. In fact, silicone sealing elements, in particular when associated with the form described and illustrated, are such as to ensure a virtually perfect seal in order to evaluate the progressive increase of the expiratory force and also determine with the required "forcing" in the nostril, and with each passing day, a dilation of the nasal thresholds generally deficitary in oral breathers.

The through-cavity 7 also crosses the sealing element 4 asymmetrically so as to position itself closer to the nasal septum and consequently well aligned with the terminal extension direction of a nostril.

In all the configurations described previously the sealing element 4 must have, in order to fulfil the sealing functions towards the maximum expiratory nasal flow and dilation of the nasal thresholds, a diameter/circumference of the widest part of flare greater than that of the nasal threshold which it is inserted in with the required "forcing". This is a peculiarity of this invention which requires sealing elements of various sizes related to the age of the patient and his nasal dimensions.

The sealing element 4 is placed at one end of the cannula 5 and the latter is substantially made from a tube for example plastic having an inner channel **8** which extends between the sealing element 4 and an open operating end **9.**

The through cavity 7 and the inner channel 8 have circular transversal cross-sections similar dimensionally and at least mainly having internal diameters between 3 and 6 millimetres.

The cannula 5 partially superposes a tubular appendage emerging from the sealing element 4, as in Fig. 4, or is pressure-inserted in the through cavity 7, as in Figs. 2a, 2b.

In Fig.1 a cannula 5 is provided for in substantially one piece, defined by a single tube preferably partially bent near the operating end 9. The cannula 5 has an end portion bent at an angle comprised between 30 ° and 60 ° with respect to its main direction of extension.

Fig. 5 instead shows a cannula 5 defined by two tubes, an upper tube **5a** and a lower tube **5b** telescopically engaged and reciprocally mobile upon a slight pressure in an axial direction.

This way the cannula 5 may assume the most convenient length for use each time, with reference to its position with respect to a patient. For example it may have a length of between about 15 and 30 cm.

A telescopic cannula is also easier to clean, especially by washing its separated parts, which individually have a very limited length.

Fig. 3 shows schematically a specific bipartite or forked cannula 5 or with double ends, so as to support two sealing elements 4, and thus to simultaneously engage both nostrils.

This is a technical solution that can be used in advanced and conclusive stages or treatment follow-up to restore proper nasal breathing.

Figures 1 and 5 show that the cannula 5 or lower tube 5b can be supported, preferably in a removable manner, by a support **11.**

The latter may have a very varied shape and is preferably provided able to support the lower portion of the cannula 5 in a substantially horizontal position or in any case in a position substantially parallel to the support surface of the support 11.

The device 1 according to the invention then comprises said second means 3 suitable to highlight the flow of air crossing - in succession - the through cavity 7 of the sealing element 4, the inner channel 8 of the tube 5, and the operating end 9.

The second means 3 are substantially consecutive to the operating end 9 or placed in correspondence therewith and comprise, in their simplest embodiment, a movable body **12** having a shape and weight suitable to allow its displacement in the presence of said air flow emitted by a nostril.

The mobile body 12 is a simple ping-pong ball and can be supported by the support 11, conveniently expanding beyond the operating end 9.

Said ball can move freely or slide towards a target or a net-not shown-distanced from the support 11, or may be conveyed inside a graduated tube or channel or path, so as to accurately highlight the maximum distance reached for the specific intensity of the impulse applied with the airflow.

The graduated tube or channel or path may be placed adjacent to or made integral with the support 11, or even made in one piece with the same.

The second means 3 may then advantageously be made in a particularly effective and functional manner by means of specific devices in addition to the mobile body 12 before described, such as shown in Fig. 6.

In said figure, number **13** indicates a flow rate meter commonly called flow meter.

This is a device in itself known and can be made using various technologies and is able to detect both the quantity and the speed of airflow. It is commonly used to measure the flow of liquids and gases, and may have a small display **13a** for example LCD to show the readings measured.

Combined with the flow meter 13 and serving it, electronic members **14** suitable to transmit signals to a receiver unit **15** defined for example by a mobile phone, a tablet, laptop or fixed PC may also advantageously be used.

The signals transmitted by said members 14 and displayed by the unit 15 relate to the flow meter readings 13. These signals may be dated, stored and processed into charts showing the progress of a treatment to stimulate and improve the results thereof.

The functioning of the device 1 described above in a structural sense, is as follows.

It implements a new procedure for improving nasal breathing, preferably by means of the device 1 described above.

Initially the device 1 should be positioned on a table or other surface, the length of the cannula 5 adjusted, if telescopic, the second means 3 prepared and a sealing element 4 inserted into a nostril, or into both.

This having been done, the exercise consists of breathing in deeply through the mouth, blowing as hard as possible and only from the nostril, or nostrils, which the sealing element 4 is inserted in, closing the mouth and opposite nostril with a finger.

In the case shown in figures 1 and 4 the airflow hits the ping-pong ball 12, or other equivalent moving body, and - proportionally to the entity of the flow - the ball is projected forward, for example towards a suitably distanced target or a net, or towards a graduated path. The greater the flow, the greater the possible distance reached or the greater the speed of the ball.

In the case shown in Figure 6 the entity of the air flow is measured, in addition, by the flow meter 13 or equivalent device.

The entity of the airflow may be instantly highlighted in a mobile phone or otherwise 15, with maximum spontaneous interest in the user of the device.

The performance of this exercise for five to ten minutes every day for a few weeks, constantly alternating one nostril with the other, allows anyone to greatly improve their nasal ventilation and general state of health of the whole body.

The cannulas with a dual end have a flow of exhaled air of half the quantity in each nostril, reducing the effectiveness of the exercise, but can still be used in specific occasions, such as when the treatment has already been substantially completed, as a simple follow-up.

The invention achieves the proposed objectives and permits important advantages.

In fact the device is effective and able to act energetically. This is because on the one hand it makes it possible to concentrate the exercises on a single nostril each time, in order to maximise the ventilation action, and on the other intercepts and conveys without any loss the exhaled air flow, and also without or with minimal pressure drops.

Use of the same thus proves simpler, more pleasant and stress-free, as it also has a recreational component and may be seen as a competition in which to constantly better one's results. As such it is suitable for use with frequency and constancy even for children of only a few years of age.

In any case it allows the user, and parents of younger patients, to directly assess and highlight the results and improvements achieved.

The device stands out for the fact of allowing maximum hygiene and a thorough cleaning of all its elements.

## Claims

1. Device (1) for improving nasal ventilation, comprising first means (2) suitable to collect a flow of air emitted from at least one nostril and second means (3) designed to highlight said flow, said first means (2) comprise:
- at least one sealing element (4) suitable to be inserted into a nostril and having internally through cavity (7),
- at least one cannula (5) having an inner duct (8) extending between said through cavity (7) and an operating end (9) open ad distanced from said sealing element (4),
- said second means (3) being substantially consecutive to said operating end (9),
- **characterized in that**:
- said sealing element (4) is suitable to be inserted in conditions of adherence into the nostril and it is a nasal olive of the type used for nasal irrigation,
- said second means (3) comprise a mobile body (12), being a ping-pong ball, having an outline and a weight such to permit its movement in the presence of said flow of air crossing said operating end (9).

2. Device according to claim 1, wherein said sealing element (4) is in silicone.

3. Device according to a previous claim, wherein said through cavity (7) said inner duct (8) have a circular cross-section and at least mainly, have internal diameters comprised between 3 and 6 mm.

4. Device according to a previous claim, in which said support (11) is provided suitable for supporting said cannula (5) on a support surface and to place at least a portion thereof in an inclined position substantially between 30° and 60° with respect to said support surface.

5. Device according to a previous claim, in which said second means (3) comprise a flow meter (13) suitable to measure characteristics of said flow.

6. Device according to a previous claim, in which said second means (3) comprise members (14) of the electronic type suitable to transmit signals related to characteristics of said flow to a unit (15) receiving and displaying said signals.

## Patentansprüche

1. Vorrichtung (1) zur Verbesserung der Nasenbeatmung, umfassend erste Mittel (2), die geeignet sind, einen aus mindestens einem Nasenloch kommenden Luftstrom aufzunehmen und zweite Mittel (3), die geeignet sind, den genannten Luftstrom zu verstärken, wobei die genannten ersten Mittel (2) Folgendes umfassen:
- mindestens ein Dichtungselement (4), das geeignet ist, anhaftend in ein Nasenloch eingesetzt zu werden und in seinem Inneren einen durchgehenden Hohlraum (7) aufweist,
- mindestens eine Kanüle (5) mit einem Innenkanal (8), die zwischen dem genannten durchgehenden Hohlraum (7) und einem geöffneten und von dem genannten Dichtungselement (4) entfernten operativen Ende (9) verläuft,
- wobei die genannten zweiten Mittel (3) im Wesentlichen auf das genannte operative Ende (9) folgen,
- **dadurch gekennzeichnet, dass**:
- das genannte Dichtungselement (4) eine Nasenolive des zur Nasenspülung verwendeten Typs ist,
- die genannten zweiten Mittel (3) einen beweglichen Körper (12) umfassen, der ein Tischtennisball ist, und eine Form und ein Gewicht aufweist, die geeignet sind, sein Verschieben beim Vorliegen des das genannte operative Ende (9) überquerenden genannten Luftstroms zu gestatten.

2. Vorrichtung (1) nach Anspruch 1, bei der das genannte Dichtungselement (4) aus Silikon besteht.

3. Vorrichtung (1) nach einem vorangegangenen Anspruch, bei der der genannte durchgehende Hohlraum (7) und der genannte Innenkanal (8) kreisförmige Querabschnitte aufweisen, die mindestens vorrangig Innendurchmesser zwischen 3 und 6 Millimetern aufweisen.

4. Vorrichtung (1) nach einem vorangegangenen Anspruch, bei der eine Halterung (11) vorgesehen ist, die geeignet ist, die genannte Kanüle (5) auf einer Ablageebene abzustützen und mindestens einen Teil derselben in einer zwischen im Wesentlichen 30° und 60° im Verhältnis zu der genannten Ablageebene geneigten Position anzuordnen.

5. Vorrichtung (1) nach einem oder mehreren der vorangegangenen Ansprüche, bei der die genannten zweiten Mittel (3) einen Durchflussmesser (13) umfassen, der geeignet ist, die Eigenschaften des genannten Luftstroms zu messen.

6. Vorrichtung (1) nach einem oder mehreren der vorangegangenen Ansprüche, bei der die genannten zweiten Mittel (3) Organe (14) elektronischen Typs umfassen, die geeignet sind, mit Eigenschaften des genannten Luftstroms verbundene Signale an ein Gerät (15) für den Empfang und das Anzeigen der genannten Signale zu übertragen.

## Revendications

1. Dispositif (1) pour améliorer la respiration nasale, comprenant des premiers moyens (2) aptes à recueillir un débit d'air émis par au moins une narine et des deuxièmes moyens (3) aptes à mettre en évidence ledit débit, lesdits premiers moyens (2) comprennent :
- au moins un élément d'étanchéité (4) apte à être inséré en condition d'adhérence dans une narine et ayant intérieurement une cavité de passage (7),
- au moins une canule (5) ayant un canal interne (8) se développant entre ladite cavité de passage (7) et une extrémité opérationnelle (9) ouverte et espacée dudit élément d'étanchéité (4),
- lesdits deuxièmes moyens (3) étant sensiblement consécutifs à ladite extrémité opérationnelle (9),
- **caractérisé en ce que** :
- ledit élément d'étanchéité (4) est un embout nasal du type utilisé pour l'irrigation nasale,
- lesdits deuxièmes moyens (3) comprennent un corps mobile (12), c'est à dire une balle de ping-pong, ayant une silhouette et un poids aptes à permettre un déplacement relatif en présence dudit débit d'air qui traverse ladite extrémité opérationnelle (9).

2. Dispositif (1) selon la revendication 1, dans lequel ledit élément d'étanchéité (4) est réalisé en silicone.

3. Dispositif (1) selon une revendication précédente, dans lequel ladite cavité de passage (7) et ledit canal interne (8) présentent des sections transversales circulaires et ayant au moins essentiellement des diamètres internes compris entre 3 et 6 millimètres.

4. Dispositif (1) selon une revendication précédente, dans lequel il est prévu un support (11) apte à soutenir ladite canule (5) sur un plan d'appui et à disposer au moins une partie de celle-ci en position inclinée essentiellement entre 30° et 60° par rapport audit plan d'appui.

5. Dispositif (1) selon une ou plusieurs revendications précédentes, dans lequel lesdits deuxièmes moyens (3) comprennent un débitmètre (13) apte à relever des caractéristiques dudit débit d'air.

6. Dispositif (1) selon une ou plusieurs revendications précédentes, dans lequel lesdits deuxièmes moyens (3) comprennent des organes (14) de type électronique aptes à transmettre des signaux liés à des caractéristiques dudit débit d'air à une unité (15) de réception et affichage desdits signaux.
